# EUROPEAN PATENT SPECIFICATION

(11) **EP 2 198 794 B1**
(45) Date of publication and mention of the grant of the patent: **25.01.2012**
(21) Application number: 09178125.2
(22) Date of filing: 04.12.2009
(51) Int. Cl.: A61B 17/74, A61B 17/70, A61F 2/82, A61F 2/90

(54) **Avascular necrosis cage manufacturing process**
Herstellungsverfahren für vaskulären Nekrosekäfig
Procédé de fabrication de cage à nécrose avasculaire

(30) Priority: 18.12.2008 US 337978
(43) Date of publication of application: 23.06.2010
(73) Proprietor: DePuy Products, Inc., Warsaw, IN 46581 (US)
(72) Inventor: Jenks, Philip, Warsaw, IN 46580 (US); Heckman, Jon M, Warsaw, IN 46580 (US); Cumberland, Mylin L, Warsaw, IN 46580 (US)
(74) Representative: Belcher, Simon James

(56) References cited:
- WO-A1-96/01599
- WO-A2-2005/102224
- WO-A2-2006/116761
- US-A1- 2002 123 750
- US-B1- 6 511 748

## Description

The present invention relates generally to a method of making a spherical cage, for example which might be implanted in a bone in the treatment of avascular necrosis.

The bone damaging disease avascular necrosis (AVN) is believed to be caused by loss of blood supply. Eventually, the bone tissue dies, causing the bone to collapse. In a hip joint, AVN can cause the collapse of the femoral head.

Most commonly, AVN is treated by an orthopaedic surgeon who performs a total hip replacement procedure. In a total hip replacement procedure, the entire hip joint is replaced with a prosthetic implant. The implant includes an acetabular cup that fits in the acetabulum, a ball for mating with the acetabular cup, and a stem that extends into the femur. However, this is a very invasive and major surgery.

It is also known to treat AVN by implanting a wire cage in the femoral head, especially using a wire formed from a nickel titanium based shape memory alloy. Implantation involves drilling a bore along the femoral neck from the anterior cortex and inserting the cage through the bore. The cage is compressed during insertion and placed inside of the femoral head. Once inside the femoral head, the cage then returns towards a spherical shape so that it reinforces the femoral head. The canal is then packed with a bone graft or a bone cement material.

WO-96/01599 discloses a method of making a medical device which involves positioning a fabric formed from a nickel-titanium shape memory alloy in a mould, deforming the fabric to confirm to the surface of the mould, heat treating the fabric to set its shape in the deformed state, and removing the fabric from the mould.

The present invention provides a method of making a hollow cage as defined in claim 1.

Preferably the former has the approximate shape of at least a part of a sphere (that is, part-spherical or wholly spherical).

In another aspect, the invention provides a rounded cage which is made by the process described above. Preferably the shape of the cage approximates to that of a sphere or at least part of a sphere.

Embodiments of the invention are described below by way of example with reference to the accompanying drawings, in which:
FIG. 1 is a perspective view of a cage positioned inside the head of a femur;
FIG. 2 is a flow chart of a method for fabricating a cage according to the invention;
FIG. 3 is a perspective view of a former in positioned within a braided wire sleeve to illustrate a step in the manufacturing process of FIG. 2;
FIG. 4 is a perspective view of another step in the manufacturing process of FIG. 2;
FIG. 5 is a perspective view of a clamping step in the manufacturing process of FIG. 2;
FIG. 6 is a disassembled view of a mould, illustrating pins that are inserted in the manufacturing process of FIG. 2;
FIG. 7 is a perspective view of the ball and cage during the manufacturing process of FIG. 2; and
FIG. 8 is a perspective view of the final cage after the manufacturing process of FIG. 2 is completed.

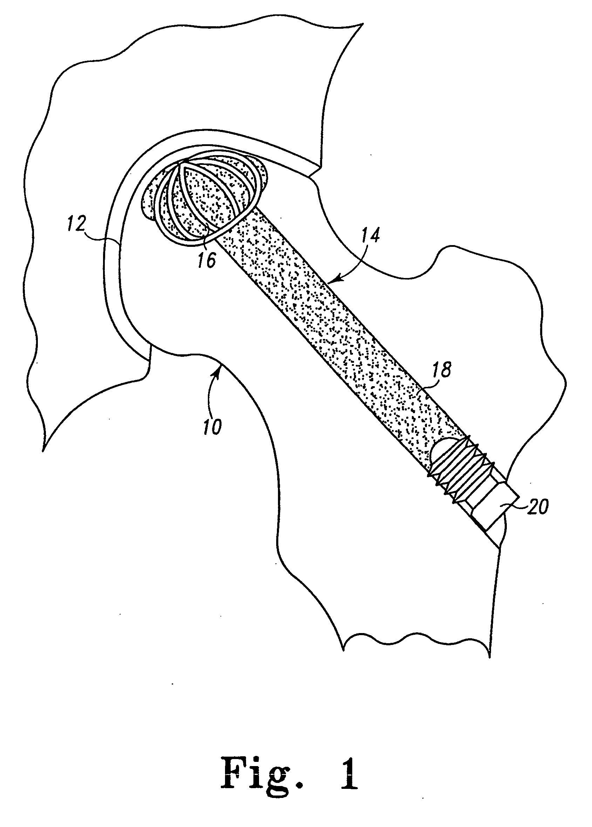

Referring to the drawings, FIG. 1 shows a femur 10 which includes a head 12 that is damaged by AVN. In order to repair the damaged head 12, a canal 14 is drilled through the femur 10 and head 12. A cage 16 is inserted through the canal 14, and provides support to the damaged head 12. Tampered bone graft 18 is used to fill in the canal 14 and any voids. A screw 20 may also be used to further plug the canal 14. The cage 16 is made of a nickel titanium based shape memory alloy (such as that known as Nitinol) which is biocompatible and which exhibits enhanced elastic properties so that it is able to be compressed during insertion through the canal and then will regain its shape. Other biocompatible materials that are compressible and that can return to their original shape after being compressed may also be used.

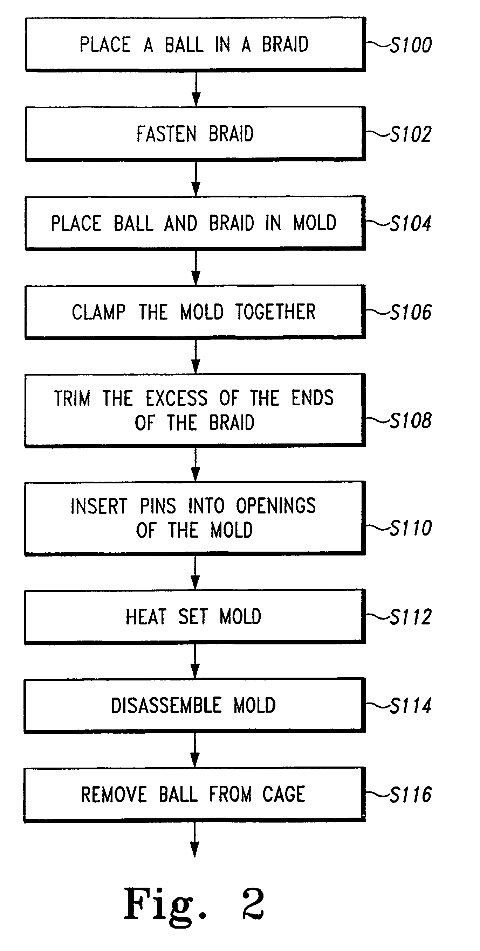

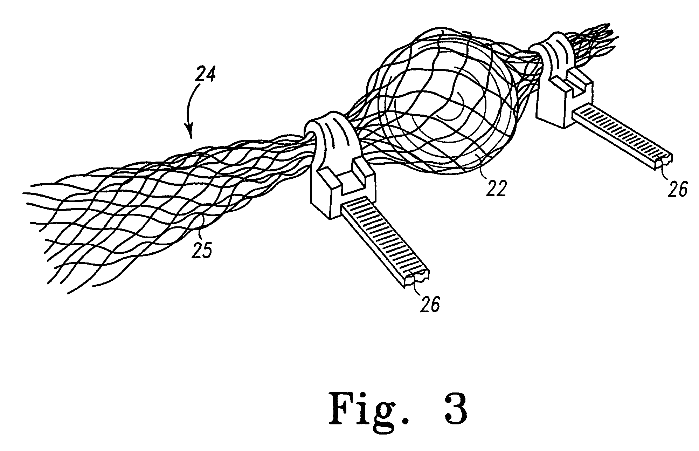

Turning now to FIG. 2, a method for manufacturing the cage 16 will be described. First, at step s100, a ball 22 is placed inside a braid 24 made of wire 25 (FIG. 3). The braid 24 is used to form the final cylindrical cage 16. In the described example, the ball is approximately spherical but the ball 22 should be selected based on the desired size and shape of the resulting cage. The ball 22 may be a spherical steel gauge ball. In other embodiments, the ball 22 may be made of other metals or ceramics. In some embodiments, the spherical ball 22 is selected from a plurality of spherical balls having a diameter ranging from about 20 mm to about 30 mm. In some embodiment, the diameters of the plurality of spherical balls are 20 mm, 25 mm, and 30 mm.

Returning now to FIG. 2, at step s102, the braid 24 is then fastened by a pair of mechanical fasteners 26 (FIG. 3). The mechanical fasteners may be ties, clamps, or other known fastening methods. In other embodiments, the braid 24 may not be clamped at all.

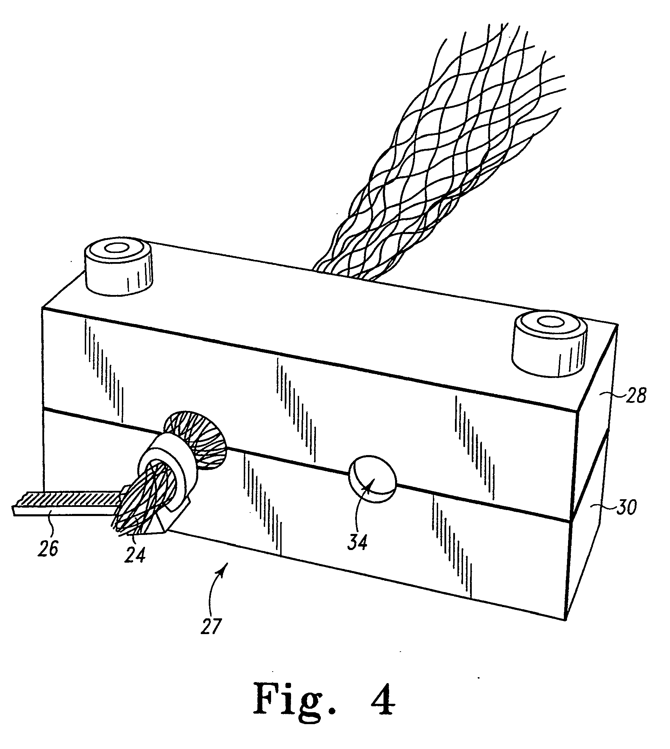

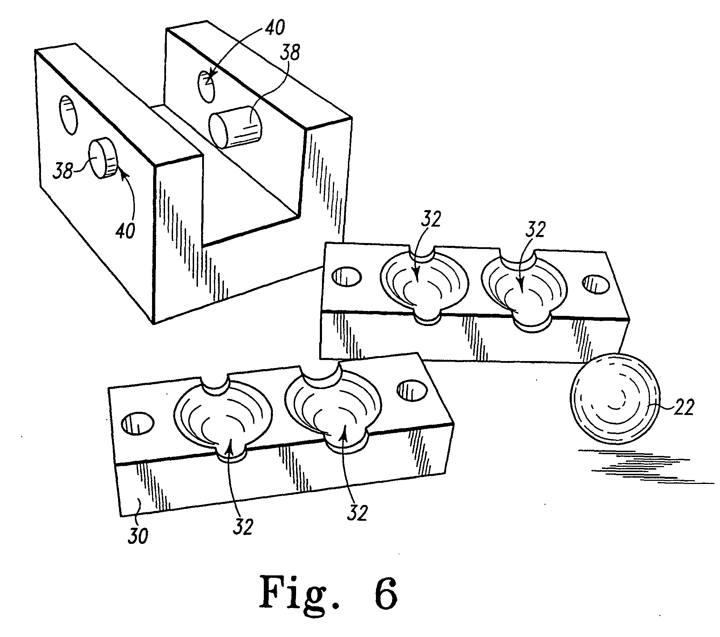

At step s104, the braid 24 and ball 22 are placed into a mould 27 (FIG. 4). The mould 27 may be made of stainless steel, other metal, or ceramic. The mould 27 includes a top half 28 and a bottom half 30. Each of the top half 28 and the bottom half 30 include a hemispherical cavity 32 that has a diameter larger than the diameter of the spherical ball 22 (FIG. 6). In some embodiments, the diameter of the hemispherical cavity is equal to the diameter of the spherical ball 22 plus four times the diameter of the wire 25. In some embodiments, the mould 27 may have more than one cavity 32 as shown in FIG. 6. If the mould 27 has a plurality of cavities 32, the cavities 32 may be of the same size to enable the manufacture of more than one cage 16 using one mould 27. Alternatively, the cavities 32 may be of differing sizes, allowing the same mould 27 to be used to make different size cages 16.

As shown in FIG. 4, the ball 22 and braid 24 are placed in the cavity 32 of the bottom half 30 of the mould 27. The top half 28 of the mould 27 is then placed on top of the bottom half 30 such that the hemispherical cavities 32 are aligned, creating a spherical cavity. The mould 27 also includes side openings 34. The side openings 34 allow for the fasteners 26 and excess braid 24 to extend out from the mould 27.

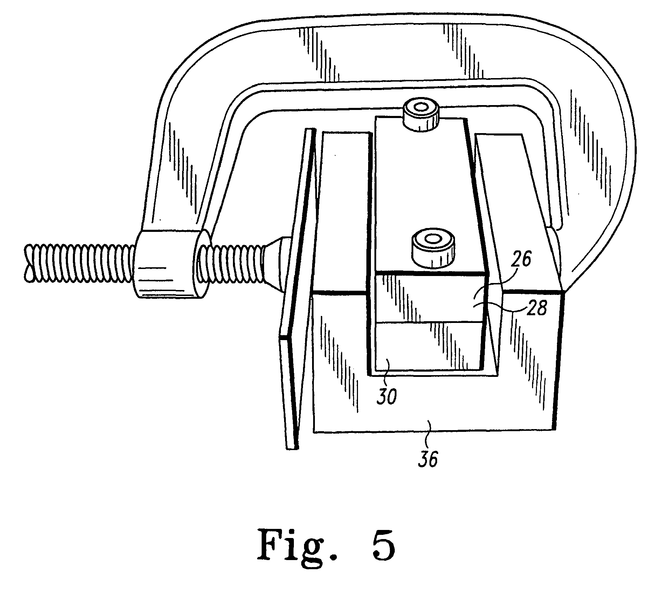

Returning to FIG. 2, at step s106, the top half 28 and bottom half 30 of the mould 27 are placed into a cradle 36 and clamped together (FIG. 5). The clamping force further presses the braid 24 into a spherical shape. At step s108, the excess braid 24 is trimmed as close to the ball as possible.

At step s110, two pins 38 (FIG. 6) are urged through side openings 40 on the cradle 36 and side openings 34 on the mould 27. The side openings 40 correspond with the side openings 34 of the mould 27 in order for the pins 38 to be urged through both sets of side openings 34, 40. The pins 38 force the trimmed ends of the braid 24 to conform to the spherical shape of the ball 22. Preferably, the two pins have the same spherical form at the end that is inserted into the side openings 34, 40, to ensure that the trimmed ends of the braid 24 are similarly shaped. In other embodiments, differently shaped ends may be used. The pins 38 may be made of stainless steel. Alternatively, the pins 38 may be made of other metals.

At step s112, the mould 27, along with the ball 22 and braid 24, are inserted into an oven and heat set. In one embodiment, the oven is set at 525°C. The mould, cage and ball were then heated for a period of three to four hours. In other embodiments, other known heat setting temperatures and times may be used.

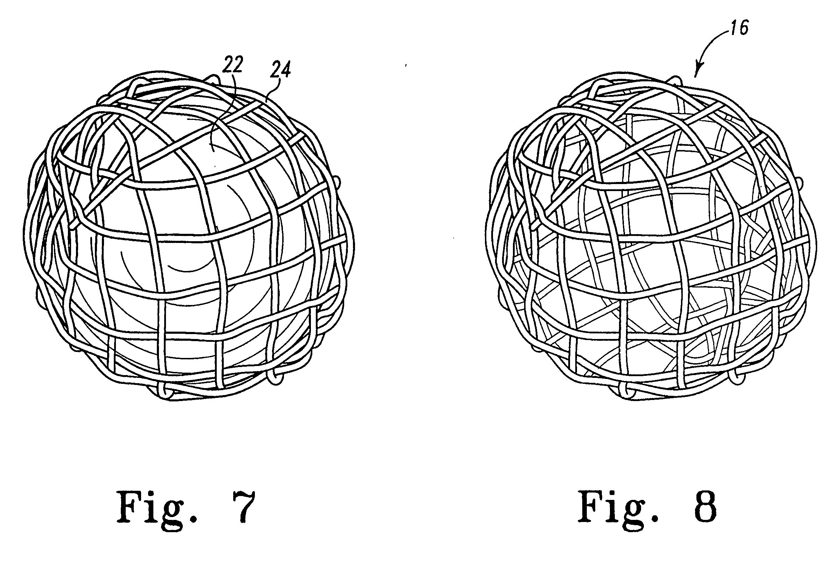

After heat setting, at step s114, the mould is dissembled and the ball 22 and braid 24 are removed (FIG. 7). Next, at step s116, the ball 22 is removed from the now spherical braid, resulting in the spherical cage 16 shown in FIG. 8. The ball 22 is squeezed out through the cage 16. The enhanced elastic properties of a shape memory alloy mean that the cage 16 can spring back towards its spherical shape.

## Claims

1. A method of making a hollow cage (16) for implantation in a bone cavity, the method comprising:
providing a wire braid (24) made from a shape memory alloy,
inserting a former (22) into the wire braid,
placing the wire braid and the former inside a mould (27) which has a pair of openings (34), one on each of two opposite sides of the mould so that, once the wire braid and the former are placed inside the mould, the excess wire of the braid on one side of the former protrudes from one of the openings and the excess wire of the braid on the opposite side of the former protrudes from the other of the openings,
heat setting the mould with the former and the wire braid,
removing the former and wire braid from the mould, and
removing the former from the wire braid, resulting in a hollow wire cage,
**characterised in that** the method includes prior to the heat setting step the step of trimming excess wire braid which extends through the openings beyond the mould so as to create trimmed ends of the braid, and the step of forcing the trimmed ends of the braid into the openings in the mould so that the trimmed ends are forced to conform to the shape of the former.

2. The method of claim 1, which includes applying fasteners (26) to the braid (24) on each side of the former (22) after the former has been placed inside the wire braid.

3. The method of claim 1, in which the trimmed ends of the braid (24) are forced into the openigns (34) in the mould (27) by placing two pins (38) through the openings.

4. The method of claim 1, in which removing the former (22) from within the braid (24) comprises squeezing former out of the braid.

5. The method of claim 1, in which the former (22) is a spherical ball.

6. The method of claim 1, in which the former (22) is formed from a metallic material or a ceramic material.

7. The method of claim 1, which includes selecting the former (22) from a plurality of formers having different sizes.

## Patentansprüche

1. Verfahren zum Herstellen eines Hohlkäfigs (16) zum Implantieren in einer Knochenhöhlung, wobei das Verfahren aufweist:
Bereitstellen eines Drahtgeflechts (24), das aus einer Formgedächtnislegierung hergestellt ist,
Einsetzen eines Formbildners (22) in das Drahtgeflecht,
Anordnen des Drahtgeflechts und des Formbildners in einer Form (27), die in Paar Öffnungen (34) hat, eine auf jeder von zwei gegenüber liegenden Seiten der Form, so dass, sobald das Drahtgeflecht und der Formbildner in der Form angeordnet sind, der überschüssige Draht des Geflechts auf einer Seite des Formbildners aus einer der Öffnungen hervorsteht und der überschüssige Draht des Geflechtes auf der gegenüber liegenden Seite des Formbildners aus der anderen der Öffnungen hervorsteht,
Thermofixieren der Form mit dem Formbildner und dem Drahtgeflecht,
Herausnehmen des Formbildners und des Drahtgeflechtes aus der Form und
Entfernen des Formbildners von dem Drahtgeflecht, was zu einem hohlen Drahtkäfig führt,
**dadurch gekennzeichnet, dass** das Verfahren, vor dem Thermofixierschritt, den Schritt des Abschneidens überschüssigen Drahtgeflechtes, das sich durch die Öffnung über die Form hinaus erstreckt, um so geschnittene Enden des Geflechtes zu erzeugen, und den Schritt des Zwingens der geschnittenen Enden des Geflechts in die Öffnungen in der Form, so dass die geschnittenen Enden gezwungenermaßen der Form des Formbildners entsprechen, umfasst.

2. Verfahren nach Anspruch 1, das das Anbringen von Befestigungselementen (26) an dem Geflecht (24) auf jeder Seite des Formbildners (22), nachdem der Formbildner in das Drahtflecht gebracht worden ist, umfasst.

3. Verfahren nach Anspruch 1, bei dem die geschnittenen Enden des Geflechtes (24) in die Öffnungen (34) in der Form (27) gezwungen werden, indem zwei Stifte (38) durch die Öffnungen geschoben werden.

4. Verfahren nach Anspruch 1, bei dem das Entfernen des Formbildners (22) aus dem Geflecht (24) das Herausquetschen des Formbildners aus dem Geflecht aufweist.

5. Verfahren nach Anspruch 1, bei dem der Formbildner (22) ein kugelförmiger Ball ist.

6. Verfahren nach Anspruch 1, bei dem der Formbildner (22) aus einem metallischen Material oder einem keramischen Material gebildet ist.

7. Verfahren nach Anspruch 1, das das Auswählen des Formbildners (22) aus einer Vielzahl von Formbildnern mit unterschiedlichen Größen umfasst.

## Revendications

1. Procédé de fabrication d'une cage creuse (16) destinée à une implantation dans une cavité osseuse, le procédé comprenant les étapes consistant à :
✔ fournir une tresse de fils (24) réalisée à partir d'un alliage à mémoire de forme ;
✔ insérer une forme (22) dans la tresse de fils ;
✔ placer la tresse de fils et la forme à l'intérieur d'un moule (27) qui présente une paire d'ouvertures (34), une sur chacun des deux côtés opposés du moule de telle sorte, une fois que la tresse de fils et la forme sont placées à l'intérieur du moule, que le surplus de fils de la tresse sur un côté de la forme fasse saillie à partir de l'une des ouvertures et que le surplus de fils de la tresse sur le côté opposé de la forme fasse saillie à partir de l'autre des ouvertures ;
✔ durcir à la chaleur le moule avec la forme et la tresse de fils ;
✔ retirer la forme et la tresse de fils du moule ; et
✔ retirer la forme de la tresse de fils, donnant une cage de fils creuse ;
**caractérisé en ce que** le procédé comprend, avant l'étape de durcissement à la chaleur, une étape consistant à couper le surplus de tresse de fils qui s'étend à travers les ouvertures au-delà du moule de façon à créer des extrémités coupées de la tresse, et une étape consistant à faire pénétrer de force les extrémités coupées de la tresse dans les ouvertures du moule de quelle sorte que les extrémités coupées soient forcées de se conformer à la forme de la forme.

2. Procédé selon la revendication 1, comprenant une étape consistant à appliquer des attaches (26) sur la tresse (24) de chaque côté de la forme (22) une fois que la forme a été placée à l'intérieur de la tresse de fils.

3. Procédé selon la revendication 1, dans lequel les extrémités coupées de la tresse (24) sont rentrées de force dans les ouvertures (34) du moule (27) en plaçant deux broches (38) à travers les ouvertures.

4. Procédé selon la revendication 1, dans lequel l'étape consistant à retirer la forme (22) hors de l'intérieur de la tresse (24) comprend une étape consistant à compresser la forme pour la faire sortir de la tresse.

5. Procédé selon la revendication 1, dans lequel la forme (22) est une boule sphérique.

6. Procédé selon la revendication 1, dans lequel la forme (22) est réalisée à partir d'un matériau métallique ou d'un matériau céramique.

7. Procédé selon la revendication 1, comprenant une étape consistant à sélectionner la forme (22) parmi une pluralité de formes qui présentent des tailles différentes.
